# EUROPEAN PATENT SPECIFICATION

(11) **EP 2 316 933 B1**
(45) Date of publication and mention of the grant of the patent: **17.06.2020**
(21) Application number: 10186250.6
(22) Date of filing: 15.09.2003
(51) Int. Cl.: C12N 5/076

(54) **Systems of sperm cell preservation and processing**
Systeme zur Spermazellenkonservierung und -verarbeitung
Systèmes de conservation et de traitement des cellules spermatiques

(30) Priority: 13.09.2002 US 410884 P; 09.01.2003 US 340881
(43) Date of publication of application: 04.05.2011
(62) Divisional of application: 03795716.4
(73) Proprietor: XY, LLC, Navasota, TX 77868 (US)
(72) Inventor: Maxwell, William, Maxwell, Chisholm, Beecroft, New South Wales 2119 (AU); Hollinshead, Fiona, Kate, Paddington, New South Wales 2021 (AU); O'Brien, Justine, Kellie, Paddington, New South Wales 2021 (AU); Evans, Gareth, Lane Cove, New South Wales 2066 (AU)
(74) Representative: Jacob, Reuben Ellis

(56) References cited:
- WO-A-01/37655
- LU K H ET AL: "IN VITRO FERTILIZATION WITH FLOW-CYTOMETRICALLY-SORTED BOVINE SPERM", THERIOGENOLOGY, LOS ALTOS, CA, US, vol. 52, no. 8, December 1999 (1999-12), pages 1393-1405, XP000995618, ISSN: 0093-691X
- SCHENK J L ET AL: "Cryopreservation of Flow-Sorted Bovine Spermatozoa", THERIOGENOLOGY, LOS ALTOS, CA, US, vol. 52, 1 January 1999 (1999-01-01), pages 1375-1391, XP002981957, ISSN: 0093-691X, DOI: 10.1016/S0093-691X(99)00224-1
- STAP J ET AL: "IMPROVING THE RESOLUTION OF CRYOPRESERVED X- AND Y-SPERM DURING DNAFLOW CYTOMETRIC WITH THE ADDITION OF PERCOLL TO QUENCH THE FLUORESCENCE OF DEAD SPERM", JOURNAL OF ANIMAL SCIENCE, AMERICAN SOCIETY OF ANIMAL SCIENCE, US, vol. 76, no. 7, 1 January 1998 (1998-01-01), pages 1896-1902, XP000985715, ISSN: 0021-8812
- HOLLINSHEAD F K ET AL: "Production of lambs of predetermined sex after the insemination of ewes with lownumbers of frozen-thawed sorted X- or Y-chromosome-bearing spermatozoa", REPRODUCTION, FERTILITY AND DEVELOPMENT, CSIRO, EAST MELBOURNE, AU, vol. 14, 1 January 2002 (2002-01-01), pages 503-508, XP008037861, ISSN: 1031-3613
- HOLLINSHEAD F K ET AL: "Sex-sorting and re-cryopreservation of frozen-thawed ram sperm for in vitro embryo production", THERIOGENOLOGY, LOS ALTOS, CA, US, vol. 59, no. 1, 1 January 2003 (2003-01-01), page 209, XP002981958, ISSN: 0093-691X
- HOLLINSHEAD F K: "In vitro and in vivo assessment of functional capacity of flow cytometrically sorted ram spermatozoa after freezing and thawing", REPRODUCTION, FERTILITY AND DEVELOPMENT, CSIRO, EAST MELBOURNE, AU, vol. 15, 1 January 2003 (2003-01-01), pages 351-359, XP008037862, ISSN: 1031-3613, DOI: 10.1071/RD03060
- HOLLINSHEAD F K ET AL: "Birth of lambs of a pre-determined sex after in vitro production of embryos using frozen-thawed sex-sorted and re-frozen-thawed ram spermatozoa.", REPRODUCTION (CAMBRIDGE, ENGLAND) MAY 2004, vol. 127, no. 5, May 2004 (2004-05), pages 557-568, XP002427618, ISSN: 1470-1626

## Description

### TECHNICAL FIELD

The present invention relates to sperm cell preservation, and the processing of sperm cells, generally. The invention may be applicable to artificial insemination, in vitro fertilization, super-ovulation, non-human mammal production, sperm samples, and sperm cell processing, including processing such as collecting, handling, sorting, storage, transportation, use, fertilization, or insemination features.

### BACKGROUND OF THE INVENTION

The fertilization of non-human mammal eggs, potentially referred to as oocytes, such as by artificial insemination and in vitro fertilization techniques have been conducted in attempt to increase the fertility rates of livestock. Furthermore, effective pre-selection of sex has been accomplished in many species of livestock following the development of safe and reliable methods of sorting, generally, or specifically separating sperm cells into enriched X chromosome bearing and Y chromosome bearing populations. Separation of X chromosome bearing sperm cells from Y chromosome bearing sperm cells, as well as collection, handling, sorting, storage, transportation, use, fertilization, or insemination techniques, or sperm cell and semen processing generally, can be accomplished as disclosed herein and as disclosed by various patent applications, for example: PCT/US99/17165; PCT/US01/45023; PCT/US01/15150; PCT/US98/27909; PCT/US01/45237, PCT/US01/18879, PCT/US00/30155, PCT/US01/02304, U.S. Patent Number 6,071,689, Patent Number 6,372,422, U.S. divisional application number 10/081,955, U.S. provisional application number 60/400,486, and U.S. provisional application number 60/400,971.

Although the various devices and methods of sperm cell processing, generally, and the collection, handling, sorting, storage, transportation, usage, fertilization, and insemination of sperm cells have been improved over the past several years, significant problems remain with respect to maintaining sperm quality, such as viability, motility, and functionality, and preservation and stimulation of sperm cells, especially with regard to artificial insemination (in vivo) and in vitro fertilization (IVF) procedures. One potential consequence is the reduction in fertilization rates. Sperm quality, such as the viability of sperm separated into enriched X-chromosome bearing and Y-chromosome bearing populations could be compared directly in-vitro (for example, in conjunction with IVF procedures) and in-vivo (for example, in conjunction with artificial insemination procedures), is generally reduced during traditional sperm cell processing. More generally, traditional processing techniques addressing sperm viability, motility, functionality, preservation, stimulation, fertilization, and insemination may have not yielded preferred or even adequate fertilization rates or sperm quality, for example.

As one example of traditional sperm processing, techniques of sperm cell sorting for the breeding of non-human mammals may be limited, for example, with regard to sperm cell quality, such as sperm cell viability, motility, and functionality, and fertilization rates as well as potentially other limited characteristics or features. For example, if the sorter apparatus is a relatively long distance from the males, or if the sorter apparatus is a relatively long distance from the receiving females to be inseminated or eggs to be fertilized with processed sperm, the sperm cell quality, fertilization rates, or other characteristics or features may be adversely affected. Some of the techniques or some traditional techniques may achieve to some degree sperm cell viability, motility, or functionality, and desirable fertilization rates, while addressing, for example, sperm preservation during transportation to the sorter apparatus. However, further techniques achieving sufficient sperm cell quality, such as viability, motility, and functionality, and other features such as sperm cell stimulation, preservation, and maintained or enhanced fertilization rates, may be desirable, especially for any one or a combination of various sperm processing steps. The processing features may include, for example, collection, handling, sorting, storage, transportation, usage, fertilization, or insemination, and especially the preservation of the sorted sperm cells from the sorter apparatus to the site of fertilization (potentially via collected individual samples or "straws"), or at any other stage of sperm processing.

As another example of the limitations of traditional sperm processing, traditional processing techniques may be limited with regard to sperm cell quality, generally, such as sperm cell viability, motility, and functionality, and sperm cell stimulation, sperm cell preservation, and fertilization rates, due to the type and the extent of processing involved. Known processing techniques such as preservation or sorting, for example, may degrade sperm quality, and further reduce fertilization rates. The degradation of sperm quality and fertilization rates may have previously proven problematic in circumstances that may have required further steps of sperm cell preservation, such as in circumstances wherein the sorter apparatus is a relatively long distance from the males or when the sorter apparatus is a relatively long distance from the receiving females to be inseminated or otherwise fertilized with processed sperm. However, the potentially identified degradation of sperm quality and fertilization rates may not have been heretofore addressed in the context of sperm cell preservation, or even identified as related to sperm cell preservation, or sperm cell processing generally, as traditional preservation techniques may have actually been understood to have contributed to the adverse effects to sperm quality, fertilization rates, or other relevant characteristics or results.

It may have been traditionally understood that additional processing steps in the processing of sperm cells or semen having a concentration of sperm cells would degrade sperm quality and reduce fertilization rates to such an extent that additional processing steps, generally, would be avoided. Specifically, it may also have been traditionally understood that processing steps such as preservation, specifically cryopreservation, and sorting might be too damaging to the sperm cells, especially in processing sequences incorporating both cryopreservation and sorting. Furthermore, it may have even been suggested and taught in traditional methods that preservation of sperm, such as cryopreservation, provided after previous processing steps so as to preserve the sperm for later procedures, such as for in vivo or in vitro techniques, could not be accomplished without compromising the sperm cells and the technique itself to such a degree that the results of the later procedures would be detrimentally affected. Accordingly, such concerns may have even been demonstrated in traditional sperm cell processing procedures, teaching away from subsequent processing steps such as sorting and cryopreservation, or processing steps incorporating multiple cryopreservation steps.

### DISCLOSURE OF THE INVENTION

The present invention may address the variety of previously identified and potentially unaddressed problems associated with reduced sperm cell quality, such as viability, motility, and functionality, and stimulation, preservation, and other sperm cell characteristics or features. Sperm cells that may have been or will be processed by one or more steps of collection, handling, sorting, storage, transportation, usage, fertilization, or insemination and potential reductions fertilization rates are further addressed. The instant invention may also address the variety of problems associated with sperm cell quality of sperm cells that have been separated into enriched X-chromosome bearing and Y-chromosome bearing populations, potentially sperm cells sorted through techniques such as flow sorting, and the potential reduction in fertilization rates. More generally, the present invention may address traditionally low fertilization rates of in vivo artificial insemination and in vitro fertilization, and sperm cell quality issues such as sperm cell viability, motility, and functionality. The present invention also addresses issues such as stimulation, preservation, and fertilization rates, as well as enhanced or maintained fertility characteristics, such as blastocyst development rate, in vivo developmental capacity, pregnancy rate, and cell division, achieving results that may have been unexpected to those skilled in the art. Additionally, the present invention addresses numbers of processed sperm potentially needed to obtain desired pregnancy rates, and further in consideration of pregnancy rates obtained respective of sorted and unsorted preserved sperm.

According to a first aspect of the invention, there is provided a method of producing a non-human mammalian sperm sample for in vitro fertilisation, including the step of obtaining sperm cells; and characterised in that the method further includes the steps of cryopreserving the sperm cells, thawing the sperm cells, sorting the sperm cells, cryopreserving the sperm cells, thawing the twice cryopreserved sperm cells; and establishing a sperm sample from the twice cryopreserved sperm cells, wherein the sperm sample is comprised of a sample selected from the group consisting of a straw, a pellet, an insemination dose, and a prepared sperm sample.

According to a second aspect, there is provided a method of producing a non-human mammalian sperm sample for artificial insemination, including the step of obtaining sperm cells; and characterised in that the method further includes the steps of cryopreserving the sperm cells, thawing the sperm cells, sorting the sperm cells, cryopreserving the sperm cells, thawing the twice cryopreserved sperm cells; and establishing a sperm sample from the twice cryopreserved sperm cells, wherein the sperm sample is comprised of a sample selected from the group consisting of a straw, a pellet, an insemination dose, and a prepared sperm sample..

### BRIEF DESCRIPTION OF THE DRAWINGS

Figure 1 is a flow diagram of one example of a sperm processing method.
Figure 2 is a flow diagram of a second example of a sperm processing method.
Figure 3 is a flow diagram of another example of a sperm processing method.
Figure 4 is a flow diagram of an example of a sperm processing method.
Figure 5 is a flow diagram of an example of a sperm processing method, potentially provided in combination with the example of Figure 1, in some examples.

### MODES FOR CARRYING OUT THE INVENTION

Semen and sperm cell processing and preservation techniques and preservation, stimulation, fertilization, and insemination addressing one or more sperm cell characteristics or sperm quality, such as sperm cell viability, motility, and functionality, and stimulation, preservation, and fertilization rates will be discussed below. Examples of enhanced or maintained fertility characteristics, such as blastocyst development rate, in vivo developmental capacity, pregnancy rate, or cell division will also be discussed. Sperm cell characteristics or sperm quality may be addressed within the context of various processing techniques, such as collection, handling, separation, storage, transportation, usage, fertilization, or insemination.

Sperm cell quality may be any one or a combination of the various attributes of sperm cells previously mentioned or further mentioned herein, such as, for example, viability, motility, and functionality and may even be considered one or a combination of fertility characteristics of the sperm cells. Sperm cell characteristic may refer to any one or a combination of various biological, chemical, physical, physiological, or functional attributes of one or more sperm cells, such as chromosome bearing attributes of the cell, or in some examples may refer to sperm cell quality as previously described.

Fertility characteristics may be enhanced or maintained, and may comprise blastocyst development rate, in vivo developmental capacity, pregnancy rate, or cell division.

Semen or sperm cell processing techniques may refer to any one or a combination of preservation, stimulation, insemination, fertilization, sorting, selection, separation, or thawing, and may be specifically directed to any one or a combination of collection, handling, selection, storage, transportation, usage, fertilization, or insemination techniques.

Sperm samples may refer to a volume containing sperm cells, potentially comprising semen, carrier fluid or other materials, and may comprise one or a plurality of pellets, straws, insemination doses or other prepared sperm samples.

Sperm cells may be maintained or enhanced by the methods described herein and provided as sperm cell preservation or, more broadly, sperm cell processing, such as through processing steps as in sperm cell sorting or sperm cell preservation, and may be embodied in accordance with fertilization and insemination techniques. Examples may comprise some component features of preservation of sperm cells or other processing techniques as may be disclosed in the previously mentioned patents and patent applications, as further described below.

Sperm cells may also be maintained or enhanced in some examples by preservation and stimulation techniques as further described below, and also in further combination with the processing, stimulation, preservation, fertilization, and insemination techniques in the previously mentioned patent applications and patents.

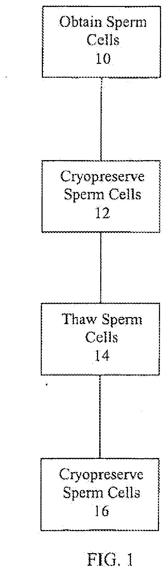

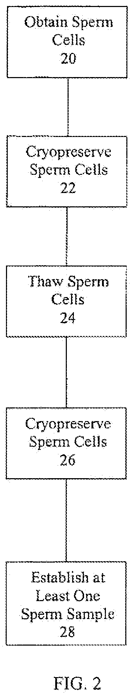

Referring to figure 1, the steps of sperm cell processing of some examples are shown in flow diagram. Sperm cells are obtained 10 and the sperm cells are cryopreserved 20. The sperm cells are then thawed 14 and a subsequent step of cryopreservation 16 is performed. Each of these features is further described below. A sperm cell processing example consistent with the features shown in Figure 1 is shown in figure 2. The example provides steps 20, 22, 24, and 26 consistent with the example of Figure 1, and further establishes at least one sperm sample. The sperm sample may comprise any sperm sample as previously described and consistent with the further description that follows.

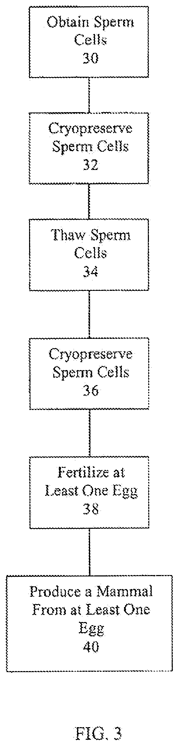

The disclosure describes a method of producing a non-human mammal, consistent with the examples of Figure 1, as shown in steps 30, 32, 34, and 36 of Figure 3. The sperm cells from feature 36 fertilize at least one egg 28 and a non-human mammal is produced 40 from the at least one egg. Again, each of these features is further described below.

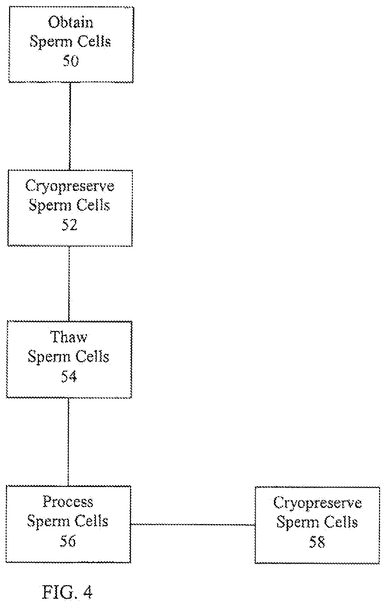

Figure 4 shows examples of sperm cell processing in accordance with the present invention. As shown, and in further elaboration on the previously described features, the sperm cells may be obtained 50 from a non-human mammalian source, either directly or in various combinations of steps, such as through a storage facility. The sperm cells may be cryoperserved 52. Cryopreservation may be provided as further described in some examples of the invention below. Subsequently, the sperm cells may be thawed 54. Further processing 56 may then be performed on the sperm cells prior to another cryopreservation of the sperm cells58.

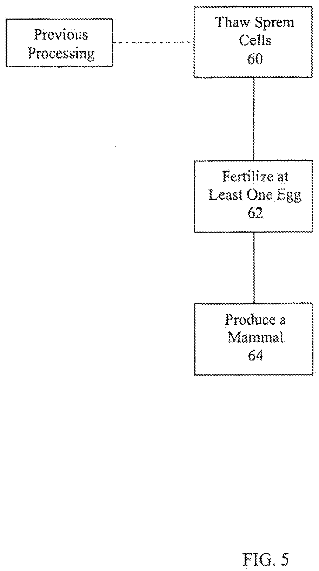

Sperm cell processing, generally, and the processes previously described may be performed independent of insemination and fertilization techniques, or as part of and in combination with such techniques. Each of the above processes may also be performed respective of super ovulation techniques. Accordingly, some examples are methods of producing a non-human mammal, as shown in Figure 5. After previous processing, or in some examples as proceeding with the features 50 through 58, at least one egg may be fertilized 62. In one example, the sperm cells may be thawed 60 and the fertilization 62 of at least one egg with the sperm cells may be conducted. A non-human mammal may then be produced 64 from the egg fertilized by the processed sperm cells. Additional processing steps such as sorting of the sperm cells is provided in some examples, and is preferably performed after the first thaw (54).

Furthermore, examples of the invention such as those previously described support in vivo and in vitro techniques. Accordingly, a female of the species of the non-human mammal from which sperm was obtained may be inseminated with the sperm cells, such as the previously processed sperm cells, or sorted and cryopreserved sperm cells, each previously described. Insemination techniques may comprise inseminating the at least one female with the sperm cells, the provision of which may be either thawed or potentially as cryopreserved sperm cells in an unthawed condition, potentially in pellet or other sperm sample configuration as previously described. Thawed sperm samples may be preferred if, for example, sperm cell quality is to be maintained or enhanced as part of the sperm cell processing or non-human mammal production. However, unthawed cryopreserved cells could be used for insemination and preferred, if, for example, a thaw of the sperm cells within the female is acceptable, and if a reduction of steps is desired outside the womb, such as to reduce sperm cells processing. Furthermore, artificial insemination and in vitro fertilization techniques, generally, in accordance with the present invention, may be accomplished with unthawed cryopreserved cells, independent of or in combination with other processing features such as thawing, subsequent steps of cryopreserving, sorting, or other features presently disclosed. Processing steps such as sorting of the sperm cells may also be performed.

Insemination may be performed with at least one sperm sample such as those previously described. At least one egg of the female may be fertilized and a non-human mammal produced from the at least one fertilized egg. In accordance with in vitro procedures, fertilizing in vitro at least one egg may be performed. The at least one fertilized egg may be transferred to a recipient female, and a non-human mammalian embryo may be produced, or in some examples, a non-human mammal may be produced, from the at least one fertilized egg.

Super ovulation techniques are also encompassed by the present invention. A female of the species of non-human mammal may be superovulated. In some examples, the superovulated female may be inseminated with the sperm cells and at least one egg fertilized. In other examples, at least one egg of the superovulated female may be obtained and the at least one egg fertilized in vitro. The at least one fertilized egg may be transferred to a recipient female in either of the artificial insemination or in vitro fertilization techniques.

As previously mentioned, methods of sperm cell processing may comprise the establishment of at least one sperm sample of the sperm cells. Accordingly, examples of the invention may comprise methods of sperm cell processing, such as those shown in Figure 2, that feature establishing at least one sperm sample of the sperm cells. In some examples, the at least one established sperm sample may be the subject of a subsequent cryopreservation step, such that the at least one sperm sample is cryopreserved.

It may have been previously thought that consistent with conventional processing techniques that such processed sperm cells, and established sperm samples, would not be of sufficient sperm quality or otherwise not be capable of fertilization. The present invention provides that the sperm sample is capable of fertilization of at least one egg of a female non-human mammal, as may be shown in the various examples described below, and further the insemination of such female with the at least one sperm sample, and in some examples, the fertilizing of at least one egg of a female non-human mammal with the at least one sperm sample. The feature of sperm samples capable of fertilization extends in some examples to capability of fertilization of a species of a superovulated female non-human mammal, and the insemination of such superovulated female with the at least one sperm sample. The feature of sperm samples capable of fertilization also extends in some examples to capability of fertilization in vitro of at least one egg, and the fertilization in vitro at least one egg with the at least one sperm sample. Further examples provide for establishing at least one sperm sample capable of inseminating at least one female non-human mammal, and the insemination of such at least one female with said at least one sperm sample. The fertilization of a plurality of eggs, and even the production of a non-human mammalian embryo, a non-human mammalian embryo that may be transferred to a recipient female, and more generally the production of a non-human mammal, such as non-human mammalian offspring, are provided consistent with the fertilization capability of the sperm sample and the additional features described above. Examples of the invention addressing sperm sample capability may further comprise producing at least one animal of a pre-selected sex from said cryopreserved processed sperm cells. Again, sperm samples may accordingly be established and may be configured as a straw, a pellet, an insemination dose, or a prepared sperm sample.

Each of the examples previously described, and those further described below, may be considered departures from traditional sperm cell processing, the production of non-human mammals, the production of non-human mammalian embryo, and the establishment of sperm samples. It has been traditionally viewed that such processing of sperm cells could not sufficiently ensure sperm quality or provide adequate rates of insemination, fertilization, or pregnancy. However, and as been taught in the various references cited herein, sperm cells may in fact be processed to achieve, for example, the sorting of sperm cells, potentially to differentiate sperm cells as either X chromosome bearing or Y chromosome bearing sperm cells, in some instances to provide for preselection of the sex of a non-human mammal or non-human mammalian embryo. The present invention also provides for such processing, and in some preferred examples, additional features of preservation heretofore traditionally thought not to be feasible commercially, or even possible, and heretofore providing a solution to those previously identified but unaddressed issues.

Semen, and in particular sperm cells, may be obtained and otherwise heretofore processed from non-human mammals in accordance with the present invention such as equids, bovids, felids, ovids, canids, buffalo, oxen, elk, or porcine, or other non-human mammal species. Further, some examples may provide obtaining and processing semen or sperm cells from prized non-human mammal species, endangered non-human mammal species, rare individuals of a non-human mammal species, and even from zoological specimens or individuals. The resulting non-human mammal or non-human mammalian embryo may be produced in accordance with the techniques as previously described and as further described below. Sperm samples may be established, in some examples, as previously described.

Sperm samples are cryopreserved, such as by freezing, using various preservation techniques, such as freezing in a Hepes-buffered crydiluent. Sperm cells may be provided as pellets or straws and may be thawed using various thawing techniques, for example, with ram spermatozoa. Other sperm samples may be provided throughout the processing of sperm cells, and may or may not be cryopreserved when established as a sperm sample or when provided to inseminate or fertilize an egg.

One or more additives, singularly or in combination, may be introduced into the semen, sperm cells, or sperm sample. In one example, a cryodiluent may be introduced into the sperm sample to preserve the sperm cells. The introduction of the additive or additives, such as a cryodiluent, into the sperm sample, and in some examples with as a cryopreservation step, potentially referred to as freezing, may maintain or enhance sperm cells, and may further maintain or enhance sperm quality, sperm cell quality, such as sperm cell viability, motility, and functionality, and may maintain or enhance stimulation and fertilization rates, and potentially one or more sperm cell characteristics. In other examples, an additive or additives, such as cryodiluent, singularly or in combination, could be removed from the sperm sample. The removal of the cryodiluent or other additives from the sperm sample, and in some examples with cryopreservation steps, may maintain or enhance sperm cells, and may further maintain or enhance sperm quality, such as maintaining or enhancing sperm cell viability, motility, and functionality, and fertilization rates, and potentially one or more sperm cell characteristics.

Sperm cells may also be maintained or enhanced in some examples of the disclosed techniques, and sperm quality may further be maintained or enhanced, such as maintaining or enhancing sperm cell viability, motility, and functionality, and potentially one or more sperm cell characteristics. Furthermore, fertilization rates may be at least maintained, and even enhanced as further described below. Fertilization characteristics may also be maintained or enhanced, and blastocyst development rates may be enhanced, monospermic fertilization at least maintained and even enhanced, and cleavage rates at least maintained and even enhanced. In some examples, the sperm sample may be preserved, as previously described, such as through cryopreservation, such as freezing, or other preservation techniques such as those disclosed in the previously mentioned patent applications. Sperm quality is maintained in the examples as further described below. Maintenance of sperm quality, however, should be construed as encompassing enhancement features associated with the sperm cells. In some examples, there may be enhanced motility, enhanced viability, and enhanced functionality of the sperm cells.

Accordingly, cryodiluent or other additives, singularly or in combination, may be introduced into the sperm sample to preserve or stimulate the sperm. In one example, the introduction of the cryodiluent or other additives into the sperm sample contributes to the preservation of the sperm through cryopreservation, freezing the sperm sample, and therefore maintaining or enhancing the sperm cells, and further maintaining or enhancing sperm quality, such as at least maintaining or even enhancing sperm cell viability, motility, and functionality, and potentially one or more sperm cell characteristics. As previously mentioned, the cryodiluent or other additives may be introduced into the sperm sample prior to sample preservation or as sample preservation. The sample may then be processed such as through collection, handling, sorting, storage, transportation, usage, fertilization, or insemination techniques as disclosed in the previously mentioned patent applications and patents.

In one example, cryodiluent or other additives, singularly or in combination, may be introduced into a sperm sample previously collected and the sample cryopreserved, such as through freezing, and followed by a thaw of the sample and subsequent processing, including collection, handling, sorting, storage, transportation, usage, fertilization, or insemination processing techniques. One such processing step may be provided as sorting, and in some examples as the separation of X chromosome bearing sperm cells from Y chromosome bearing sperm cells, potentially into a high purity population sample or samples. Various benefits may be achieved through such a sperm processing technique, including the maintenance of sperm quality. For example, various methods of sorting as described in the previously mentioned patent applications and patents achieve a separation of X chromosome bearing sperm cells from Y chromosome bearing sperm cells while minimizing damage to the viable sperm cells. Further, non-viable sperm, contamination, or cryodiluent or other additives, for example, may be eliminated through such separation techniques. Additionally, other aspects of sperm quality may be maintained or enhanced, particularly that of sperm cell viability, motility and functionality. The sperm sample may further be stimulated during the processing to maintain or enhance sperm quality as previously described. One such method of sperm processing known in the art is described in U.S. Patent No. 5,135,759, disclosing a flow cytometer sorting technique.

The processing of sperm cells may be performed by sorting the sperm cells as previously described, or in some examples, in accordance with the following process. Sorting may comprise, in some examples, as a selecting of sperm cells based upon at least one desired characteristic, potentially sperm cell quality characteristics, such as viability, motility, functionality, stimulation, and preservation, or one or a combination of various sperm cell characteristics such as biological, chemical, physical, physiological, or functional attributes of one or more sperm cells, such as chromosome bearing attributes of the cell. The sperm cells may be stained, preferably with Hoechst 33342 or like stain or dye, or in combination with such stains or dyes, and the sperms cells differentiated based upon the staining and selection. The cells may then be separated based upon the differentiation and then collected. The sperm cells may be so processed in accordance with the various techniques of those references cited herein and in some examples, processed to so comprise the maintenance of sperm quality.

### EXAMPLE 1

In one example of a preserving and processing technique, 200µl of thawed spermatozoa was placed onto a 2ml separation gradient (90%:45%) of Puresperm™, a human preparation, and a Tris based diluent. The gradient preparations were then centrifuged at 1000g for 15 minutes. The post-Puresperm™ pellet was removed, slowly diluted 1:4 with warm Tris based diluent and centrifuged at 650g for 3 minutes. The supernatant was removed and the sperm stained, incubated and sorted as previously described. The Tris based diluent was used as the staining medium and Androhep® (Minitub, Germany) plus 20% egg yolk was used as the collection medium.

The above example is one of various examples of the inventive technique, providing preservation of sperm, such as through cryopreservation or freezing, thawing the sperm, identifying the sperm, such as through staining, and sorting the sperm, such as into X and Y chromosome bearing populations.

Although the previous example provides a sperm preservation technique and processing technique of sperm collection, preservation, thaw and separation, other techniques may be used. For example, one or a combination of various collection, handling, separation, storage, transportation, usage, fertilization, or insemination techniques may be performed as part of this present inventive technique. In one example, sperm may be collected, followed by separation of X chromosome bearing sperm cells from Y chromosome bearing sperm cells. A preservation step prior to sperm cell separation may not be needed, for example, during circumstances in which the sorter apparatus is readily available after sperm collection. The sorted sperm sample or samples may then be preserved as an additional step and as previously described, potentially for further handling, separation, storage, transportation, usage, fertilization or insemination. The sorted sperm sample may further be stimulated during the processing to maintain or enhance sperm quality as previously described. The next example describes one example of a processing technique.

### EXAMPLE 2

Sorted samples were centrifuged at 700g for 6 min. at room temperature (24°C). The supernatant was removed and the sorted sperm could be used "fresh" for AI or in an IVF system; or the remaining pellet was extended 1:4 with the Hepes based cryodiluent, potentially the same diluent used for an original cryopreservation of the sperm, and frozen using various techniques, such as those previously described and as described below. The re-frozen and sorted sperm were thawed using methods such as a glass tube shaken in a 37°C waterbath, and then could be used in an AI or IVF system.

### EXAMPLE 3

The use of spermatozoa sorted from frozen-thawed pellets in the ovine mammal regarding in vitro fertilization systems. Frozen-sorted and frozen-sorted-frozen sperm used in an ovine IVF system were slowly diluted with 0.5ml of IVF media, and in some examples, using ovine IVF protocol, and centrifuged in a Falcon tube with a tight lid at 300g for 6 minutes. The supernatant was removed and the remaining sperm quickly placed in the IVF well at a concentration of one million motile sperm/ml. Preferably, a high standard of media preparation (i.e. use of eggs less than 24 hours old, ultracentrifugation of egg yolk diluents and meticulous filtering) and handling of the samples (i.e. constant temperature) is required.

Other preservation, processing, fertilization, and insemination techniques need not include a separation step. For example, the sperm may be collected, followed by a preservation step and subsequent handling, storage, transportation, usage, fertilization, or insemination.

Furthermore, one or more preservation steps may be conducted as part of preservation, processing, fertilization, and insemination techniques, or combinations thereof, such preservation in some examples comprising cryopreservation, such as through freezing of the sperm sample, and subsequent steps of thawing, occurring before, concurrent with, or after one or more other processing techniques.

### EXAMPLE 4

Application of sperm sorting to breeding of livestock and wildlife may be limited when the sorter is a long distance from the male(s), as previously described, but would be facilitated by the sorting of cryopreserved and thawed sperm, such as frozen-thawed sperm (Lu KH et al., Theriogenology 1999:52:1393-1405) and cryopreserving or refreezing it. High purity sorting with maintained quality of frozen-thawed ram sperm may be achieved after processing to remove the cryodiluent. The aim of this study was to evaluate the functional capacity of frozen-thawed sperm after sorting and a second cryopreservation/thawing step. Frozen semen from 2 rams (n=2 ejaculates per ram) was used throughout. Post-thaw sperm treatments comprised (i) unsorted (Control); (ii) sorted (Frozen-Sort) and (iii) sorted then re-frozen (Frozen-Sort-Frozen). X and Y sperm were separated using a high-speed sorter (SX MoFlo(R), Cytomation, CO, USA) after incubation with Hoechst 33342 and food dye to eliminate non-viable sperm. Reanalysis revealed high levels of purity for X- and Y-enriched samples for all treatments (87.0 +/-4.5%). For IVF, 472 IVM oocytes were inseminated with 1 x 10(6) motile sperm/mL. After 3 h in SOF medium, oocytes were transferred to Sydney IVF cleavage medium (Cook(R), QLD, Australia) for 4 d followed by Sydney IVF blastocyst medium (Cook(R)) for an additional 3 d culture in 5% O2: 5%CO2: 90% N2. Oocytes were assessed for cleavage at 24 and 48 h post-insemination (p.i.). At 52 h p.i., uncleaved oocytes were stained with orcein for assessment of maturation and fertilization. Data from 3 replicates were analyzed by ANOVA, Chi-square and Fisher Exact Test. At insemination, % motile sperm (+/- SEM) was higher (P<0.001) for Frozen-Sort (85.8 +/- 2.4%) and Frozen-Sort-Frozen (66.7 +/- 7.7%) than Control (36.7 +/- 2.1%). Maturation rate was 95.6% (451/472). Cleavage of oocytes in a parthenogenetic control group (no sperm) was low (2/56; 3.6%). Polysperminc fertilization was low (9/451; 2.0%) and did not differ among treatments.

**Table 1. Fertilization & early embryo development of oocytes after incubation with frozen-thawed unsorted (Control), frozen-thawed & sorted (Froz-Sort) & frozen-thawed, sorted then frozen-thawed (Froz-Sort-Froz) ram sperm. Values in parentheses are percentages.**

| Treatment | No. of mature oocytes fertilized^{d} | No. of mature oocytes undergoing cleavage after insemination | | No. of cleaved oocytes forming blastocysts | | |
|---|---|---|---|---|---|---|
| | | 24h | 48h | Day 5 | Day 6 | Day 7 |
| Control | 40 (67.8) | 26 (44.1) | 36 (61.0) | 4 (11.1) | 13 (36.1) | 16 (44.4)^{a} |
| Froz-Sort | 110 (63.6) | 67 (38.7) | 109 (63.0) | 24 (22.1) | 34(31.2) | 57 (52.3)^{ab} |
| Froz-Sort-Froz | 94 (57.7) | 71 (43.6) | 91 (55.8) | 23 (25.3) (64.8)^{bc} | 347(40.7) | 59 |

| | | | | | | |
|---|---|---|---|---|---|---|
| ^{d}Monospermic fertilization. Within column, values with different superscripts differ (P<0.05). | | | | | | |

Fertilization and cleavage rates were consistently high across treatments. Blastocyst development rate was higher for oocytes fertilized with Froz-Sort-Froz than with Control sperm. These results demonstrate that frozen-thawed ram sperm can be sex-sorted for either immediate or future use in an IVF system after re-cryopreservation.

The above example is one of various examples of the inventive technique, providing preservation of sperm, such as through cryopreservation, such as freezing, thawing the sperm, sorting the sperm, such as into X and Y chromosome bearing populations, preserving the sorted sample, such as through cryopreservation or freezing, and further including thawing the sperm sample for use, such as for fertilization or insemination.

Consideration should be given to fertility rates respective of preservation and processing procedures, such as separation for sex-sorting and cryopreservation of sperm samples, as the next example describes, and in combination with cryopreservation, thawing, processing, and subsequent cryopreservation, as disclosed in this description.

### EXAMPLE 5

Lambs have been produced after artificial insemination (AI) with low numbers (2-4x10⁶) of cryopreserved sex-sorted sperm. Fewer ewes were pregnant after AI with X- or Y-sorted frozen-thawed (25%, 15% respectively) than with a commercial dose of unsorted frozen-thawed sperm (54%). The object of the present study was to determine the minimum numbers of sorted frozen-thawed sperm required to obtain pregnancy rates similar to those obtained with unsorted sperm.

A sample of sperm from single ejaculates of 2 rams was stained, incubated, analyzed and sorted using a modified high speed cell sorter (MoFlo®, Cytomation, Fort Collins, CO, USA) as previously described. Sperm were processed at 15,000-18,000/serc without sex-sorting into 10ml centrifuge tubes pre-soaked with 1% BSA in sheath fluid containing 0.2ml Tris-buffered medium and 20% egg yolk (v/v). For every sample, 1.3 x 10⁶ sperm were sex-sorted and analyzed to determine purity. Sorted and unsorted (control) samples were extended with a zwitterions-buffered diluent containing 13.5% egg yolk and 6% glycerol and frozen as 250ul pellets containing 5x10⁶ sperm. The time of estrus was controlled in 144 Merino ewes by progestagen sponges (FGA, Vetrepharm A/Asia, Sydney) inserted intravaginally for 12 days and an injection of 400 I.U of PMSG (Pregnecol, Vetrepharm A/Asia) at sponge removal (SR). Thirty-six h after SR 134 ewes were injected with 40µg GnRH (Fertagyl®, Intervet) to control the time of ovulation. One hundred and eleven ewes were inseminated in the uterus by laparoscopy 57-60h after SR with 5, 10, 20 or 40x10⁶ sorted or unsorted frozen-thawed sperm. Thirteen ewes not given GnRH were inseminated with a commercial dose of unsorted frozen-thawed sperm. Thirteen ewes not given GnRH were inseminated with a commercial dose of unsorted frozen-thawed sperm 57-58h after SR. Pregnancy was diagnosed by ultrasound on d53. The data were analyzed by Chi-square.

Sperm motility after thawing was 37.8+/-1.78% (sorted) and 42.9+/-0.93% (unsorted). Seven of 13 (53.8%) ewes not given GnRH were pregnant. Of the GnRH-treated ewes the proportion pregnant was affected by the number of sperm inseminated (p<0.05) but not by ram or type of sperm (p>0.05). For ewes inseminated with sorted or unsorted (control) frozen-thawed sperm, pregnancy rate was higher for inseminates of 10 and 40x10⁶ than for 5 and 20x10⁶ sperm (Table 1). The results suggest that a minimum of 40x10⁶ sorted frozen-thawed sperm inseminated close to the time of ovulation are required to obtain commercially acceptable pregnancy rates.

**Table 1: Pregnancy after intrauterine insemination of ewes with frozen-thawed control and sorted ram sperm.**

| Dose (x10⁶ sperm) | No. ewes inseminated | No. ewes pregnant | % ewes pregnant |
|---|---|---|---|
| 5 | 30 | 10 | 33.3^{a} |
| 10 | 28 | 16 | 57.1^{b} |
| 20 | 29 | 10 | 34.5^{a} |
| 40 | 23 | 16 | 69.6^{a} |

| | | | |
|---|---|---|---|
| ^{ab}Within columns different superscripts differ (p<0.05). ^{a}This research was supported by XY, Inc; CO, USA; The Australian Research Council, Vetrephann A/Asia. | | | |

### EXAMPLE 6

The ability to sort and re-freeze frozen-thawed sperm would significantly increase the potential application of sperm processing technology such as sperm sexing technology to applications such as species management. Frozen-thawed, sorted, re-frozen then thawed ram sperm, in accordance with some examples , appear fully functional in vitro with blastocyst production greater than that for frozen-thawed, non-sorted sperm. (Hollingshead FK et al. 2003 Theriogenology 59 209). Some examples may be especially useful to evaluate the in vitro capacity of in vitro produced non-human embryos derived from frozen-thawed sperm after sorting and a second cryopreservation/thawing step.

Frozen semen from 2 rams (n=3 ejaculates per ram) was used throughout one technique. Post-thaw sperm treatments comprised (i) non-sorted (Control); (ii) sorted (Froz-Sort) and (iii) sorted then re-frozen (Froz-Sort-Froz). X and Y sperm were separated using a high-speed sorter (SX MoFlo®, DakoCytomation, Fort Collins, CO, USA) after incubation with Hoechst 33342 and food dye to eliminate non-viable sperm. Reanalysis in some examples revealed high levels (mean ± SEM) of purity for X- and Y-enriched samples for all treatments (89 ± 1.2%). At Day 6 post-insemination in one performed technique, 2 embryos (blastocyst stage or greater) were transferred per recipient. Data were analyzed by Chi-square and Fisher Exact Test. In vivo embryo survival for this example may be similar across sperm treatments (28/64, 43.8% overall) and 20 of 23 (87.0%) sexed lambs were of the predicted sex (Table 2). These results of examples may demonstrate high in vivo developmental capacity of in vitro produced sexed embryos derived from frozen-thawed ram sperm after sorting and a second cryopreservation/thawing step, and increase the potential application of sperm processing technology such as sexing technology.

**Table 2: In vivo survival of transferred in vitro produced embryos derived from frozen-thawed non-sorted (Control), frozen-thawed & sorted (Froz-Sort) & frozen-thawed, sorted then frozen-thawed (Froz-Sort-Froz) ram sperm.**

| Sperm treatment | No. recipients | No. pregnant on Day 20 (%)^{a} | No. pregnant on Day 60 (%)^{b} | No. pregnancies lost between Day 20 and Day 60 (%) | No. lambs born/ no. of transferred embryos (%) |
|---|---|---|---|---|---|
| Control | 8 | 6 (75.0) | 5 (62.5) | 1 (16.7) | 5/16 (31.3) |
| Froz-Sort | 9 | 7 (77.8) | 5 (55.6) | 2 (28.6) | 6/18 (33.3) |
| Froz-Sort-Froz | 17 | 14 (82.4) | 14 (82.4) | 0 (0) | 17/34 (50.0) |

| | | | | | |
|---|---|---|---|---|---|
| Diagnostic by blood progesterone assay. Diagnosed by ultrasonography. | | | | | |

Methods can further include a sperm sample, as previously defined, such as a straw, and in some examples straws utilized in IVF, produced in accordance with any of the above described examples, such as any of the processing, production of non-human mammal, production of non-human mammal embryo, stimulation, preservation, fertilization, and insemination techniques, and be of maintained or enhanced sperm quality, such as a straw of desired viability, motility, and functionality, or other characteristics, or combinations of characteristics, such as characteristics disclosed herein, potentially resulting in desirable levels of fertilization rates, and in some examples, particularly for equine mammals. The sperm sample such as at least one or a plurality of straws may be particularly suited for individual production of non-human embryos.

Methods can further include a non-human mammal produced in accordance with any of the above described examples, or can include a non-human mammal of predetermined sex in accordance with the various examples that provide sperm cell insemination samples having an enriched population of either X-chromosome bearing sperm cells or enriched population of Y-chromosome bearing sperm cells, or a non-human mammal produced in accordance with any example in which a sperm cell insemination sample containing a low number of sperm cells compared to the typical number used to inseminate that particular species of non-human mammal is used, or elk progeny produced in accordance with the examples as described above.

The examples further includes various processing, preservation, stimulation, fertilization, insemination, and non-human mammal or non-human mammal embryo production techniques as disclosed herein and as may comprise in combination features disclosed in the previously mentioned patent applications and references. Accordingly, the various semen and sperm cell processing features provided as systems of preservation, stimulation, fertilization, insemination, and non-human mammal or non-human mammal embryo production examples, address sperm quality such as one or more sperm cell characteristics, such as viability, motility, functionality, or fertilization rates consistent with the disclosures of the previously mentioned patent applications and references. Further, sperm cell characteristics may be addressed within the context of various collection, handling, separation, storage, transportation, usage, fertilization, or insemination techniques, and in those or other various examples, within the context of assaying, testing, or determining the biological, chemical, physical, physiological, or functional attributes of sperm cells. Therefore, systems may provide sperm cell processing, stimulation, preservation, fertilization, and insemination, for example, incorporating flow sorting techniques, high purity separation techniques, low dose fertilization and insemination techniques, heterospermic insemination procedures, such as to assess comparative viability, motility, function, or fertility processed in various pressure environments within a sorter, as but a few examples.

## Claims

1. A method of producing a non-human mammalian sperm sample suitable for in vitro fertilisation, including the step of:
obtaining sperm cells; and **characterised in that** the method further includes the steps of:
cryopreserving the sperm cells;
thawing the sperm cells;
sorting the sperm cells;
cryopreserving the sperm cells;
thawing the twice cryopreserved sperm cells; and
establishing a sperm sample from the twice cryopreserved sperm cells; wherein the sperm sample is comprised of a sample selected from the group consisting of a straw, a pellet, an insemination dose, and a prepared sperm sample.

2. The method of producing a non-human mammalian sperm sample suitable for in vitro fertilisation as claimed in claim 1, **characterised in that** the sperm sample is capable of fertilizing at least one egg, in vitro.

3. The method of producing a non-human mammalian sperm sample suitable for in vitro fertilisation as claimed in any one of the preceding claims **characterized in that** the twice cryopreserved sperm cells are suitable for producing at least one animal of a pre-selected sex.

4. A method of producing a non-human mammalian sperm sample suitable for artificial insemination, including the step of:
obtaining sperm cells; and **characterised in that** the method further includes the steps of:
cryopreserving the sperm cells;
thawing the sperm cells;
sorting the sperm cells;
cryopreserving the sperm cells;
thawing the twice cryopreserved sperm cells; and
establishing a sperm sample from the twice cryopreserved sperm cells; wherein the sperm sample is comprised of a sample selected from the group consisting of a straw, a pellet, an insemination dose, and a prepared sperm sample.

5. The method of producing a non-human mammalian sperm sample as claimed in claim 4, **characterised in that** the sperm sample is suitable for producing at least one animal of a pre-selected sex.

## Patentansprüche

1. Verfahren zur Erzeugung einer nicht-menschlichen Säugerspermaprobe, die für In-vitro-Fertilisation geeignet ist, das den folgenden Schritt einschließt:
Erhalten von Spermazellen; und **dadurch gekennzeichnet ist, dass** das Verfahren weiter die folgenden Schritte einschließt:
Kryokonservieren der Spermazellen;
Auftauen der Spermazellen;
Sortieren der Spermazellen;
Kryokonservieren der Spermazellen;
Auftauen der zweimal kryokonservierten Spermazellen; und
Erstellen einer Spermaprobe aus den zweimal kryokonservierten Spermazellen;
wobei die Spermaprobe aus einer Probe besteht, die aus der Gruppe ausgewählt ist, die aus einem Halm, einem Pellet, einer Besamungsdosis und einer aufbereiteten Spermaprobe besteht.

2. Verfahren zur Erzeugung einer nicht-menschlichen Säugerspermaprobe, die für In-vitro-Fertilisation geeignet ist, nach Anspruch 1, das **dadurch gekennzeichnet ist, dass** die Spermaprobe mindestens eine Eizelle in vitro fertilisieren kann.

3. Verfahren zur Erzeugung einer nicht-menschlichen Säugerspermaprobe, die für In-vitro-Fertilisation geeignet ist, nach einem der vorstehenden Ansprüche, **dadurch gekennzeichnet, dass** die zweimal kryokonservierten Spermazellen für die Erzeugung von mindestens einem Tier eines vorausgewählten Geschlechts geeignet sind.

4. Verfahren zur Erzeugung einer nicht-menschlichen Säugerspermaprobe, die für die künstliche Besamung geeignet ist, das den folgenden Schritt einschließt:
Erhalten von Spermazellen; und **dadurch gekennzeichnet ist, dass** das Verfahren weiter die folgenden Schritte einschließt:
Kryokonservieren der Spermazellen;
Auftauen der Spermazellen;
Sortieren der Spermazellen;
Kryokonservieren der Spermazellen;
Auftauen der zweimal kryokonservierten Spermazellen; und
Erstellen einer Spermaprobe aus den zweimal kryokonservierten Spermazellen;
wobei die Spermaprobe aus einer Probe besteht, die aus der Gruppe ausgewählt ist, die aus einem Halm, einem Pellet, einer Besamungsdosis und einer aufbereiteten Spermaprobe besteht.

5. Verfahren zur Erzeugung einer nicht-menschlichen Säugerspermaprobe nach Anspruch 4, das **dadurch gekennzeichnet ist, dass** die Spermaprobe für die Erzeugung von mindestens einem Tier eines vorausgewählten Geschlechts geeignet ist.

## Revendications

1. Méthode de production d'un échantillon de sperme mammalien non humain convenable pour une fertilisation *in vitro,* comportant l'étape consistant à :
obtenir des cellules de sperme ; et **caractérisée en ce que** la méthode comporte en outre les étapes consistant à :
cryoconserver les cellules de sperme ;
décongeler les cellules de sperme ;
trier les cellules de spermes ;
cryoconserver les cellules de sperme ;
décongeler les cellules de sperme cryoconservées deux fois ; et
établir un échantillon de sperme à partir des cellules de sperme cryoconservées deux fois ; dans laquelle l'échantillon de sperme est composé d'un échantillon choisi dans le groupe constitué par une paillette, un culot de centrifugation, une dose d'insémination, et un échantillon de sperme préparé.

2. Méthode de production d'un échantillon de sperme mammalien non humain convenable pour une fertilisation *in vitro,* selon la revendication 1, **caractérisée en ce que** l'échantillon de sperme est capable de fertiliser au moins un ovule, *in vitro.*

3. Méthode de production d'un échantillon de sperme mammalien non humain convenable pour une fertilisation *in vitro,* selon l'une quelconque des revendications précédentes, **caractérisée en ce que** les cellules de sperme cryoconservées deux fois sont convenables pour la production d'au moins un animal d'un sexe présélectionné.

4. Méthode de production d'un échantillon de sperme mammalien non humain convenable pour une insémination artificielle, comportant l'étape consistant à :
obtenir des cellules de sperme ; et **caractérisée en ce que** la méthode comporte en outre les étapes consistant à :
cryoconserver les cellules de sperme ;
décongeler les cellules de sperme ;
trier les cellules de spermes ;
cryoconserver les cellules de sperme ;
décongeler les cellules de sperme cryoconservées deux fois ; et
établir un échantillon de sperme à partir des cellules de sperme cryoconservées deux fois ; dans laquelle l'échantillon de sperme est composé d'un échantillon choisi dans le groupe constitué par une paillette, un culot de centrifugation, une dose d'insémination, et un échantillon de sperme préparé.

5. Méthode de production d'un échantillon de sperme mammalien non humain, selon la revendication 4, **caractérisée en ce que** l'échantillon de sperme est convenable pour la production d'au moins un animal d'un sexe présélectionné.
